(19) Europäisches Patentamt
European Patent Office
Office européen des brevets

(11) **EP 2 063 842 B1**

(12) **EUROPÄISCHE PATENTSCHRIFT**

(45) Veröffentlichungstag und Bekanntmachung des Hinweises auf die Patenterteilung:
**09.11.2016 Patentblatt 2016/45**

(21) Anmeldenummer: **07802355.3**

(22) Anmeldetag: **19.09.2007**

(51) Int Cl.:
*A61F 13/515* (2006.01)    *B29C 65/08* (2006.01)

(86) Internationale Anmeldenummer:
**PCT/EP2007/008134**

(87) Internationale Veröffentlichungsnummer:
**WO 2008/034593 (27.03.2008 Gazette 2008/13)**

(54) **HYGIENEARTIKEL ODER OP-ABDECKUNGSARTIKEL ODER OP- BEKLEIDUNGSARTIKEL ZUM EINMALIGEN GEBRAUCH**

DISPOSABLE HYGIENE ARTICLE, SURGICAL COVERING ITEM, OR SURGICAL GARMENT

ARTICLE HYGIÉNIQUE JETABLE OU RECOUVREMENT CHIRURGICAL OU VÊTEMENT CHIRURGICAL JETABLE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **22.09.2006   DE 102006046420**

(43) Veröffentlichungstag der Anmeldung:
**03.06.2009   Patentblatt 2009/23**

(73) Patentinhaber: **Paul Hartmann Aktiengesellschaft**
**89522 Heidenheim (DE)**

(72) Erfinder:
• **FRANK, Bernd**
  **89555 Steinheim (DE)**
• **KESSELMEIER, Rüdiger**
  **89542 Herbrechtingen (DE)**
• **KOCH, Christian**
  **89429 Bachhagel (DE)**
• **GRÖNER, Rudolf**
  **89555 Söhnstetten (DE)**

(74) Vertreter: **DREISS Patentanwälte PartG mbB**
**Postfach 10 37 62**
**70032 Stuttgart (DE)**

(56) Entgegenhaltungen:
EP-A- 0 295 957          EP-A- 0 657 153
WO-A1-2004/105668        US-A- 5 226 992
US-A- 5 667 609          US-B1- 6 713 159

EP 2 063 842 B1

**Beschreibung**

Die Erfindung betrifft einen Hygieneartikel zum einmaligen Gebrauch, wie Inkoritinenzwindel, Inkontinenzvorlage, mit den Merkmalen des Oberbegriffs des Anspruchs 1.

Beispielsweise werden im Bereich der Hygieneartikel, im Besonderen bei Inkontinenzprodukten, wie z.B. Inkontinenzwindeln, seitliche im Gebrauch zum Benutzer aufstehende Barrieremittel, sogenannte Cuffelemente, vorgesehen, die eine Vliesstoffkomponente umfassen, die in Produktlängsrichtung auf einer weiteren Vliesstoff- oder Folienkomponente des Hygieneartikels durch Verschweißung mittels Ultraschall oder durch Verklebung fixiert werden. Allgemein ist auf dem hier in Rede stehenden Gebiet der Hygieneartikel die Verbindung von Vliesstoff- und/oder Folienkomponenten mittels Ultraschallschweißen bekannt. Ein derartiger Hygiene artikel ist beispielweise in WO-2004/05668 beschrieben. Beispielsweise kann ein Fügemuster, welches diskrete Ultraschallschweißstellen umfasst, in einer kontinuierlich, also endlos arbeitenden Herstellungsmaschine für Vliesstoffartikel der vorstehend genannten Art dadurch realisiert werden, dass eine in der Maschinenrichtung abrollende Konturwalze und ein in Schwingungen des Ultraschallbereichs versetzter als Sonotrode bezeichneter Amboss verwendet wird. Konturwalze und Sonotrode bilden einen Spalt, durch den die zu fügenden schweißbaren Komponenten während der Endlosherstellung durchgeführt werden. Durch Übertragung der Schwingungsenergie an den erhabenen Bereichen der Konturwalze erfolgt dann die Verschweißung der Vliesstoff- oder Folienkomponenten. Die Stärke der Verschweißung kann über den Abstand zwischen Sonotrode und Konturwalze und über die Steuerung der Schwindung der Sonotrode reguliert werden.

Die Anmelderin hat festgestellt, dass gerade bei schnelllaufenden Herstellungsmaschinen die Erreichung einer gleichbleibend sicheren Verschweißung ohne Beschädigung der Komponenten sich dann als äußerst problematisch erweist, wenn die Fläche der Verschweißung in der Maschinenrichtung stark variiert, da dies dann eine sehr hochfrequente Variierung der Ansteuerung der Schweißvorrichtung bzw. deren Sonotrode erforderlich macht. Es ist äußerst schwierig bis nahezu unmöglich, einen stabilen Betriebszustand zu erreichen. Wenn hingegen die Fläche eines Fügemusters in der Maschinenrichtung nicht oder nur wenig variiert, wenn es also beispielsweise in Umfangsrichtung der Konturwalze nicht variiert und beispielsweise aus konzentrischen Prägelinien oder Prägepunkten auf der Konturwalze besteht, so tritt das vorstehend geschilderte Problem nicht auf, da sich die verschweißte Fläche dann in Maschinenrichtung nicht rasch ändert. Wenn hingegen das Fügemuster zur Maschinenrichtung geneigt ausgebildet ist oder hierzu geneigte Abschnitte aufweist, so ändert sich der Anteil der verschweißten Fläche in der Maschinenrichtung sehr stark, und es kommt zu den vorstehend geschilderten Problemen. Wenn beispielsweise bei einem einzelnen Produkt das Fügemuster bogenförmige Bereiche mit enger Krümmung zur Maschinenrichtung aufweist, so besteht das Problem einer prozesssicheren Verschweißung der Vliesstoffkomponenten. Wird dieses Problem nicht beherrscht, so kommt es zu unverschweißten Stellen oder aber zu Beschädigungen der Vliesstoff- oder Folienkomponenten durch Überschweißen. In beiden Fällen ist die Gebrauchsfunktion des herzustellenden Artikels gefährdet. Extrem kurze Regelzyklen im Hinblick auf die Ansteuerung der Ultraschallschweißvorrichtung können auch zur Übersteuerung der Vorrichtung und somit häufig zu direktem Kontakt von Konturwalze und Sonotrode führen. Dies führt aber zu Beschädigungen der Vorrichtung, was deren Lebensdauer deutlich vermindert. Sonotrode und Konturwalze bedürfen dann infolge hohen Verschleißes durch unmittelbaren Kontakt der beiden Maschinenbestandteile der häufigen Erneuerung.

Der vorliegenden Erfindung liegt die Aufgabe zugrunde, einen Hygieneartikel der eingangs beschriebenen Art zur konzipieren, bei dem die vorausgehend geschilderten Probleme nicht auftreten, wobei gleichwohl ein in der Maschinenrichtung variierendes Fügemuster aus diskreten Ultraschallschweißstellen herstellbar sein soll. Es soll also möglich sein, beispielsweise aufstehende Cuffelement nach einem zur Maschinenrichtung bogenförmig schwingenden Fügemuster zu fixieren oder generell schweißbare als Flachmaterial zugeführte Vliesstoff- oder Folienkomponenten der eingangs genannten Artikel miteinander zu verbinden, wobei von der exakt geradlinigen in Maschinenrichtung erstreckten Fügung abgewichen werden können soll. Insbesondere soll eine Beschädigung der miteinander zu verschweißenden Komponenten während des Schweißprozesses sowie eine partiell mangelhafte Verschweißung der Komponenten ausgeschlossen werden. Ebenso soll eine Beschädigung der Ultraschallschweißvorrichtung vermieden werden und eine hohe Lebensdauer auch in Verbindung mit schnelllaufenden Herstellungsmaschinen sichergestellt werden.

Diese Aufgabe wird durch einen Hygieneartikel mit den Merkmalen des Anspruchs 1 gelöst. Die Lösung der Aufgabe erfolgt durch eine Auslegung des Fügemusters der Ultraschallschweißstellen derart, dass in der Maschinenrichtung die von der Verschweißung erfasste Fläche nicht so stark variiert, dass es zu den vorstehend geschilderten Problemen kommt. Dies kann beispielsweise durch Variierung und entsprechende Anordnung der Anzahl der Ultraschallschweißstellen pro Fläche, also auch durch entsprechende Wahl des Abstands der Ultraschallschweißstellen voneinander und/oder durch Variation der jeweiligen Fläche der Ultraschallschweißstellen erreicht werden.

Der genannte Index der Schwankung der von den Ultraschallschweißstellen erfassten Fläche wird folgendermaßen ermittelt: Der anspruchsgemäße Artikel wird in der ersten Richtung in 5 mm breite Längsabschnitte unterteilt betrachtet. Diese Längsabschnitte erstrecken sich also über 5 mm in der ersten Richtung, die auch der Maschinenrichtung entspricht, und senkrecht hierzu in der zweiten Richtung, die der Produktquerrichtung entspricht. Jeder dieser in der Längsrichtung 5 mm erstreckte Abschnitt umfasst eine Anzahl von Ultraschallschweißstellen, von denen jede einen verschweißten Flächenanteil bildet. Von jedem 5 mm-abschnitt lässt sich die verschweißte Fläche $A_i$ ermitteln (Summe der Flächen der einzelnen Ultraschallschweißstellen in einem 5 mm-Abschnitt). Weiter lässt sich ein Mittelwert $\overline{A}$ der von den Ultraschallschweißstellen jedes Abschnitts erfassten Fläche $A_i$ ermitteln. Beispielsweise lässt sich ein in der ersten Richtung 820 mm langer Artikel in 164 solcher 5 mm-Abschnitte unterteilen. Für jeden Abschnitt lässt sich die verschweißte Fläche $A_i$ ermitteln und aus diesen 164 Werten $\overline{A}$ lässt sich dann der arithmetische Mittelwert $\overline{A}$ der verschweißten Fläche der 5 mm-Abschnitte ermitteln. Hieraus lässt sich ein Wert s nach folgender Formel ermitteln:

$$ s = \sqrt{\frac{1}{N-1}\sum_{i=1}^{N}(A_i - \overline{A})^2} $$

Es wird also die Summe der Abweichungsquadrate der $A_i$ der einzelnen 5 mm-Abschnitte vom Mittelwert $\overline{A}$ der Schweißflächen der 5 mm-Abschnitte gebildet und durch (N-1) geteilt und aus dem Ergebnis die Quadratwurzel gezogen. Hiervon ausgehend ist der vorstehend erwähnte Index I (in %) der Schwankung der von den Ültraschallschweißstellen erfassten Fläche wie folgt definiert:

$$ I = \frac{s}{\overline{A}} \times 100[\%] $$

Es hat sich gezeigt, dass der beanspruchte Bereich des Index der Schwankung der von den Ultraschallschweißstellen erfassten Fläche von höchstens 40% im Besonderen bei hier interessierenden Maschinengeschwindigkeiten von 100 m/min bis 1000 m/min, insbesondere von 150 m/min bis 700 m/min geeignet ist, eine prozessstabile Ausführung der Ultraschallschweißverbindung zwischen Vliesstoffkomponenten und/oder Folienkomponenten der hier in Rede stehenden Artikel zu gewährleisten. Als besonders vorteilhaft erweist es sich, wenn der Index der Schwankung der von den Ultraschallschweißstellen erfassten Fläche höchstens 35%, insbesondere höchstens 30% und weiter insbesondere höchstens 25% beträgt (Anspruch 2).

Wie bereits erwähnt, handelt es sich bei einem Cuffelement um ein aus einem Flachmaterial gebildetes Seiten-Barrieremittel, welches sich zumeist durch Elastifizierung, insbesondere in Richtung Körper des Benutzers, von der Unterlage abhebt und so eine Auslaufsperre, zumeist zu den Seiten des Artikels hin bewirkt. Dieses Cuffelement ist bei einem erfindungsgemäßen Artikel durch ein erfindungsgemäß ausgebildetes Fügemuster von Ultraschallschweißstellen an weitere (Chassis-) Vliesstoff- oder Folienkomponenten des Artikels gefügt.

Die weitere Vliesstoff- oder Folienkomponente ist das Topsheet oder das Backsheet, so dass das Cuffelement mit seiner ersten Vliesstoffkomponente durch das erfindungsgemäß konzipierte Fügemuster von Ultraschallschweißstellen an dem Topsheet oder Backsheet festgelegt ist.

Zur Anfügung eines Cuffelements durch Ultraschallschweißen an weitere Komponenten des Artikels erweist es sich als vorteilhaft, dass Fügemuster ein erstes Teilfügemuster umfasst, welches zumindest bereichsweise den Cuffsockel bildet, also einen Bereich, entlang dessen das Cuffelement an die weitere Vliesstoffkomponente oder Folienkomponente angefügt ist. Dieser bildet dann bestimmungsgemäß eine Schwenklinie entlang der sich das Cuffelement beim bestimmungsgemäßen Gebrauch von einer Basisebene weg erhebt.

Der Cuffsockel ist zusätzlich bereichsweise von dem zweiten im Wesentlichen geradlinig und insbesondere im Wesentlichen parallel zu der ersten Richtung verlaufenden Teilfügemuster gebildet oder begrenzt. Unter einem im Wesentlichen geradlinigen Teilfügemuster ist hier und im Folgenden dann die Rede, wenn über eine Länge von 200 mm in der ersten Richtung eine Ausdehnung in der zweiten Richtung von maximal 6 mm feststellbar ist.

Der Cuffsockel wird zumindest abschnittsweise gebildet von dem ersten Teilfügemuster, dem zweiten Teilfügemuster und einem dritten nicht geradlinig verlaufenden Teilfügemuster. Das zweite Teilfügemuster ist in der ersten Richtung vorzugsweise zwischen dem ersten und dem dritten Teilfügemuster angeordnet. Es ergibt sich so also eine Ausbildung des Cuffsockels, der in einem vorderen Bereich und in einem hinteren Bereich des Artikels quer zur Längsrichtung, bogenförmig geschwungen hierzu verläuft und in einem dazwischen angeordneten Bereich im Wesentlichen geradlinig und insbesondere parallel zur ersten Richtung verläuft. Eine solche Anordnung erweist sich gerade im Hinblick auf Hygieneartikel zum einmaligen Gebrauch, wie Inkontinenzwindeln, Inkontinenzvorlagen als besonders vorteilhaft. Die Erfindung eröffnet hier die Möglichkeit, die Cuffelemente in einem vorderen und hinteren Bereich des Artikels in Querrichtung verhältnismäßig weit außen anzuordnen und dann in Richtung auf den Schrittbereich nach innen schwenkend auszubilden. Im Schrittbereich verlaufen die Cuffelemente bzw. die Cuffsockel oder Cuffsockelinien dann in vorteilhafter Weise im Wesentlichen parallel zueinander und haben einen geringeren Abstand in der Querrichtung (zweite Richtung) voneinander als in den Vorder- und Rückenbereichen. Mit der vorliegenden Erfindung wurde es möglich, eine stabile Verbindung zwischen Cuffelement und Chassismaterialien eines Hygieneartikels herzustellen, die den gewünschten Konfigurationsanforderungen an den Artikel gerecht wird und dennoch bei Beachtung der erfindungsgemäßen Auslegung des Fügemusters prozesssicher herstellbar ist.

In weiterer Ausbildung der Erfindung erweist es sich als vorteilhaft, wenn bei einem erfindungsgemäßen Artikel das Fügemuster in der ersten Richtung betrachtet virtuell in drei gleich lange Abschnitte, nämlich einen Anfangsabschnitt, einen sich daran anschließenden Mittelabschnitt und einen sich an den Mittelabschnitt anschließenden Endabschnitt unterteilbar ist und dass der Index der Schwankung der von den Ultraschallschweißstellen erfassten Fläche des Anfangsabschnitts und/oder des Endabschnitts des Fügemusters höchstens 40%, insbesondere höchstens 35%, weiter insbesondere höchstens 30%, weiter insbesondere höchstens 25% beträgt. Gemäß dieser weiteren Ausführungsform der Erfindung wird die erfindungsgemäß formulierte Bedingung nicht nur über den gesamten beanspruchten Artikel unter Betrachtung aller 5 mm-Abschnitte, in denen Ultraschallschweißstellen vorgesehen sind, erfüllt, sondern auch für die entsprechenden 5 mm-Abschnitte des ein Drittel überfangenden Anfangsabschnitts und des ein Drittel überfangenden Endabschnitts erfüllt.

Wie bereits erwähnt, kann die erfindungsgemäße Auslegung des Fügemusters der Ultraschallschweißstellen durch verschiedene Maßnahmen erreicht werden. Insbesondere kann es sich als vorteilhaft erweisen, wenn der Abstand der diskreten Ultraschallschweißstellen voneinander in einem ersten Bereich geringer als in einem zweiten Bereich ist.

Insbesondere erweist es sich als zweckmäßig, dass der Abstand einzelner (diskreter) Ultraschällschweißstellen voneinander in einem ersten Bereich 0,1 - 2,5 mm, insbesondere 0,2 - 1,5 mm und weiter insbesondere 0,2 - 1,0 mm beträgt und in einem zweiten Bereich 2 - 20 mm, insbesondere 3 - 15 mm, weiter insbesondere 4 - 10 mm beträgt.

Es ist auch denkbar, dass die Flächendichte der Ultraschallschweißstellen (Anzahl pro Flächeneinheit) in einem ersten Bereich größer ist als in einem zweiten Bereich.

Nach einer weiteren Ausfuhrungsform der Erfindung umfasst das erste und/oder dritte Teilfügemuster eine Schar aus mindestens zwei, insbesondere mindestens drei Kurven, weiter insbesondere mindestens vier, weiter insbesondere mindestens fünf Kurven.

Zweckmäßigerweise weisen mindestens zwei der Kurven einer Schar einen geringen maximalen Abstand von 2 - 20 mm, insbesondere 2 - 15 mm und weiter insbesondere 3 - 10 mm auf. Weter erweist es sich als vorteilhaft, wenn außerdem mindestens zwei der Kurven einer Schar einen größeren maximalen Abstand von 10 - 70 mm, insbesondere 15 - 60 mm und weiter insbesondere 20 - 50 mm aufweisen. Dies ist beispielsweise dann möglich und vorteilhaft, wenn die Schar aus drei Kurven besteht und der maximale Abstand der ersten äußeren Kurve zur mittleren Kurve den zuvor genannten geringen Wert aufweist und der maximale Abstand der zweiten äußeren Kurve zur mittleren Kurve den größeren Abstand aufweist. Es versteht sich, dass auch bei einer Schar aus mehr als drei Kurven jeweils mindestens zwei Kurven einer Schar einen unterschiedlichen maximalen Abstand von einander aufweisen können.

Des Weiteren erweist es sich als vorteilhaft, wenn eine oder mehrere der Kurven einen Radius von mindestens 60 mm, insbesondere mindestens 70 mm, weiter insbesondere 80 mm, weiter insbesondere mindestens 90 mm, weiter insbesondere mindestens 100 mm und weiter insbesondere höchstens 150 mm aufweisen. Die Kurven können auch einen veränderlichen Abstand voneinander aufweisen.

Bei den Kurven handelt es sich in vorteilhafter Weise nicht um eine entlang ihrer Erstreckung durchgehende Schweißlinie, sondern um eine Reihe von aufeinander folgend angeordneten Ultraschallschweißstellen, die dann im Wesentlichen linienförmig ist und so die Kurve bildet. Durch Variation des Abstands der Ultraschallschweißstellen und/oder durch Veränderung des Abstands der Kurven voneinander lässt sich der Flächenanteil der Schweißstellen je 5 mm-Abschnitt variieren, damit insgesamt der beanspruchte Indexbereich eingehalten wird.

Es erweist sich als vorteilhaft, wenn das Fügemuster erste diskrete Ultraschallschweißstellen mit einer Flächengröße von 0,3 - 4 mm², insbesondere 0,3 - 3 mm², weiter insbesondere 0,3 - 2 mm², weiter insbesondere 0,5 - 1,1 mm² und weiter insbesondere 0,7 - 0,9 mm² aufweist.

Des Weiteren kann es sich - wie bereits angedeutet - als vorteilhaft erweisen, wenn das Fügemuster erste und zweite diskrete Ultraschallschweißstellen aufweist und die Flächengröße der ersten diskreten Ultraschallschweißstellen kleiner ist als die der zweiten diskreten Ultraschallschweißstellen.

Die flächenhafte Ausbildung der Ultraschallschweißstellen ist eher nicht kritisch; sie können kreisförmig oder oval oder dreieckig oder mehreckig oder sichelförmig oder strichpunktförmig oder sternförmig oder linienförmig sein. Beispielsweise bei der Herstellung von Hygieneartikeln kann es sich als vorteilhaft erweisen, wenn die Summe $(A_i)$ der Flächen der Ultraschallschweißstellen eines in der ersten Richtung 5 mm langen Abschnitts des Fügemusters durchschnittlich 2 - 25 mm², insbesondere 3 - 18 mm², insbesondere 4 - 12 mm² und weiter insbesondere 6 - 10 mm² beträgt. Bei dieser Betrachtung eines Hygieneartikels mit beidseitigen Cuffelementen wird vorzugsweise jeweils lediglich das Fügemuster eines der Cuffelemente herangezogen.

Des Weiteren erweist es sich als vorteilhaft, wenn die Summe $(A_i)$ der Flächen der Ultraschallschweißstellen eines, insbesondere jedes in der ersten Richtung 5 mm langen Abschnitts des Fügemusters höchstens 30 mm², insbesondere höchstens 23 mm², weiter insbesondere höchstens 18 mm², weiter insbesondere höchstens 16 mm² weiter insbesondere höchs-

tens 15 mm$^2$ beträgt.

Bei der Herstellung eines erfindungsgemäßen Artikels erweist es sich bei der Konzeption des Fügemusters der Ultraschallschweißstellen als vorteilhaft, wenn die Abweichung der Summe ($A_i$) der Flächen der Ultraschallschweißstellen eines jeden in der ersten Richtung 5 mm langen Abschnitts des Fügemusters von der Summe ($A_i$) der Flächen der Ultraschallachweißstellen eines direkt benachbarten 5 mm langen Abschnitts des Fügemusters weniger als 70 %, insbesondere weniger als 60 %, insbesondere weniger als 50 %, weiter insbesondere weniger als 40 % beträgt. Dies bedeutet also, dass die jeweils gesamte verschweißte Fläche ($A_i$) zweier nebeneinander angeordneter 5 mm-Abschnitte sich höchstens um die genannten Bereiche unterscheiden dürfen. Hierbei wird diese Abweichung zweier benachbarter 5 mm langer Abschnitte auf die Summe $A_i$ der Flächen der Ultraschallschweißstellen desjenigen Abschnitts bezogen, dessen Summe $A_i$ der Flächen der Ultraschallschweißstellen den höheren Wert aufweist.

Des Weiteren erweist es sich im Hinblick auf eine gleichbleibende Produktqualität als vorteilhaft, wenn die Abweichung der Summe der Flächen der Ultraschallschweißstellen von mindestens 50 % insbesondere von mindestens 60 %, weiter insbesondere von mindestens 70 % und weiter insbesondere von mindestens 80 % und ganz besonders von mindestens 90 % der 5 mm langen Abschnitte des, Fügemusters von der Summe ($A_i$) der Flächen der Ultraschallschweißstellen eines direkt benachbarten 5 mm langen Abschnitts des Fügemusters weniger als 40 %, insbesondere weniger als 30 %, insbesondere weniger als 25 % beträgt.

Die Erfindung betrifft auch ein Verfahren zur Herstellung eines Hygieneartikels oder OP-Abdeckungsartikels oder OP-Bekleidungsartikels der erfindungsgemäßen Art mit den Merkmalen der Ansprüche 7, 8 und 9.

Des weiteren erweist es sich als vorteilhaft, wenn der beanspruchte Artikel so ausgebildet ist, dass das Fügemuster sich 300 - 2000 mm, insbesondere 350 - 1500 mm, insbesondere 400 - 1200 mm, weiter insbesondere 450 - 1100 mm und weiter insbesondere 500 - 1000 mm in einer ersten Richtung und senkrecht dazu vorzugsweise 10 - 1200 mm, insbesondere 15 - 1000 mm, insbesondere 20 - 500 mm, weiter insbesondere 30 - 200 mm und weiter insbesondere 40 - 150 mm und weiter insbesondere 50 - 130 mm in einer zweiten Richtung erstreckt.

Weitere Merkmale, Einzelheiten und Vorteile der Erfindung ergeben sich aus den beigefügten Patentansprüchen und aus der zeichnerischen Darstellung und nachfolgenden Beschreibung der Erfindung anhand von erläuternden Beispielen. In der Zeichnung zeigt:

Figur 1    eine Draufsicht auf einen Hygieneartikel in Form einer Inkontinenzwindel in eben äusgefaltetem Zustand;

Figur 2    eine Schnittansicht mit Schnittebene II-II in Figur 3;

Figuren 3, 4    eine teilweise perspektivische Ansicht einer Ultraschallschweißvorrichtung zur Herstellung erfindungsgemäßer Artikel in einem endlosen Fertigungsprozess unter Verwendung von endlos zugeführten Flachmaterialien;

Figur 5    eine vergrößerte Ausschnittsdarstellung eines aus Figuren 5, 6 ersichtlichen Fügemusters (betrachtet in Draufsicht gemäß Pfeil V auf die Vorrichtung nach Figur 4;

Figur 6    eine Draufsicht auf ein erfindungsgemäß ausgebildetes Fügemusters zur Verdeutlichung der gedanklichen Unterteilung in 5 mm- Abschnitte;

Figur 7    ein Diagramm, in dem der Flächenanteil der Ultraschallschweißstellen eines jeweiligen 5 mm-Abschnitts aufgetragen und das zugrunde liegende Fügemuster angedeutet ist (nicht vom Schutzbereich der Erfindung umfasst); und

Figur 8    ein entsprechendes Diagramm mit einer erfindungsgemäß ausgebildeten Fügemusterstruktur.

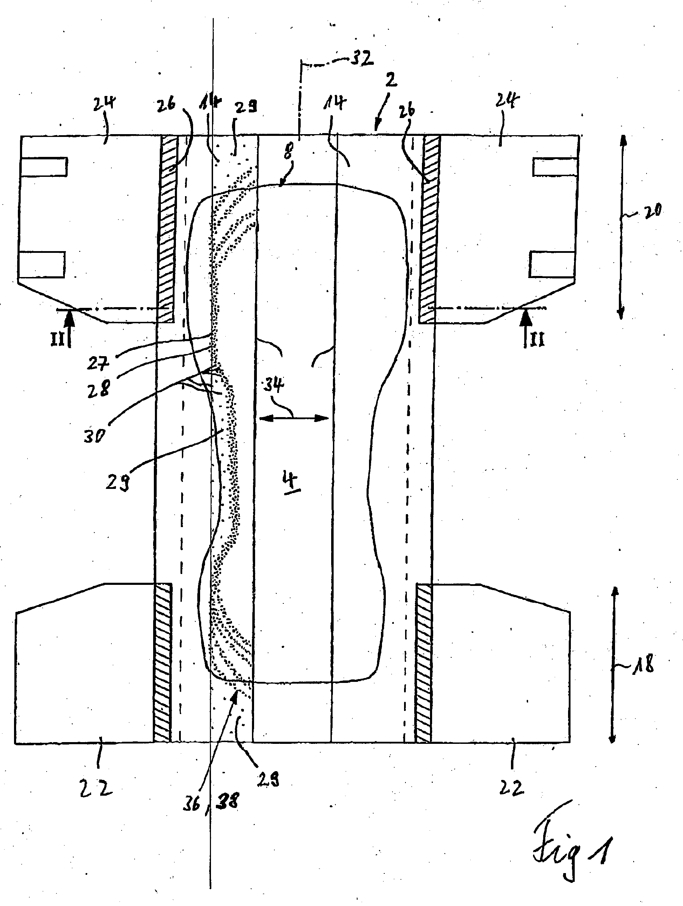

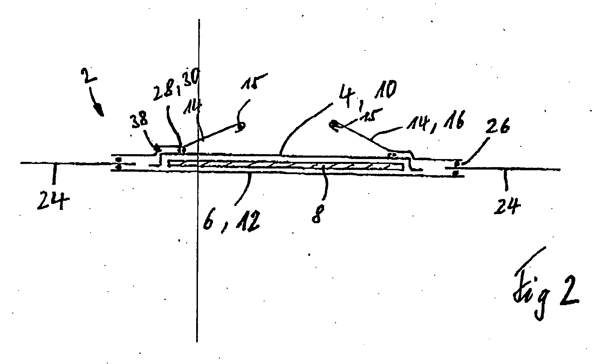

Figur 1 zeigt einen insgesamt mit dem Bezugszeichen 2 bezeichneten Hygieneartikel in Form einer Inkontinenzwindel zum einmaligen Gebrauch. Figur 2 stellt eine schematische Schnittansicht mit Schnittebene II-II in Figur 1 dar. Der Hygieneartikel 2 umfasst ein Topsheet 4 und ein flüssigkeitsdichtes Backsheet 6 sowie einen dazwischen angeordneten Absorptionskörper 8. Bei dem Topsheet 4 handelt es sich um eine flüssigkeitsdurchlässige Vliesstoffkomponente, insbesondere um ein Karden- oder Spinnvlies, vorzugsweise mit einem Flächengewicht von 8-30 g/m$^2$, insbesondere 12-25 g/m$^2$, insbesondere 14-22 g/m$^2$ und bei dem Backsheet 6 um eine flüssigkeitsundurchlässige Folienkomponente oder um ein Vlies-/Folienverbundmaterial 12. Des Weiteren umfasst der Hygieneartikel, wie am besten aus Figur 2 ersichtlich ist, seitliche Cuffelemente 14 mit an deren distalem Rand angeordneten, im vorgespannten Zustand daran vorzugsweise durch Schmelzhaftkleber festgelegten, fadenförmigen Elastifizierungsmitteln (15), die einen seitlichen Auslaufschutz bilden. Auch diese Cuffelemente (14) umfassen vorzugsweise eine Vliesstoffkomponente 16, insbesondere ein hydrophobes Spinnvlies oder ein Spunbond-Meltblown- (SM) oder ein Spunbond-Meltblown-Spunbond- (SMS) Vlieslaminat mit einem Flächengewicht von 8-30 g/m$^2$, insbesondere 12-20 g/m$^2$, insbesondere 13-18 g/m$^2$.

Jeweils in einem Vorderbereich 18 und in einem Rückenbereich 20 sind zu den Seiten erstreckte Seitenklappen 22 bzw. 24 vorgesehen. Die Seitenklappen 22, 24 erstrecken sich zwischen Topsheet 4 und Backsheet 6 und sind dort durch Schweißen oder Kleben festgelegt. Eine Schweiß- oder Klebelinie ist mit Bezugszeichen 26 angedeutet.

Außerdem sind die beidseitigen Cuffelemente 14 in einem ersten Bereich 27 entlang eines sogenannten Cuffsockels 28, sowie in einem zweiten Bereich 29 mit der Vliesstoffkomponente 10 des Topsheets 4 durch ein Fügemuster bildende diskrete Ultraschallschweißstellen 30 verbunden. Die diskreten Ultraschallschweißstellen 30 weisen in dem ersten Bereich 27 einen maximalen Abstand voneinander von 0,1 mm - 2,5 mm und in dem zweiten Bereich 29 einen maximalen Abstand voneinander von 4,0 mm - 10,0 mm auf und besitzen eine Flächengröße von jeweils 0,7 - 0,9 mm$^2$. Der Verlauf des Cuffsockels 28 ist am besten aus Figur 1 ersichtlich. Nur der Übersichtlichkeit halber wurde in Figur 1 auf die Darstellung des zweiten Fügemusters, welches das rechte Cuffelement an das Topsheet 4 festlegt, verzichtet. Das nicht dargestellte zweite Fügemuster ist spiegelbildlich angeordnet.

Denkbar und vorteilhaft wäre in einer hier nicht dargestellten Ausführungsform, den Cuffsockel 14 durch das Fügemuster direkt an dem Backsheet 6 festzulegen. Dies wäre insbesondere dann vorteilhaft, wenn der Cuffsockel 14 außerhalb der Saugkörperkontur und außerhalb der Quererstreckung des Topsheets 4 verläuft.

Der Hygieneartikel 2 umfasst des Weiteren eine erste Richtung 32, welche die Längsrichtung des Hygieneartikels bildet und bei der Herstellung des Hygieneartikels mit der Maschinenrichtung übereinstimmt. Bei der Endlosfertigung erstrecken sich also die entsprechenden Flachmaterialbahnen bildenden Topsheet 4, Backsheet 6, Seitenklappen 24 und Cuffelemente 14 in dieser ersten Richtung 32. Die Erstreckung des Cuffsockel 28 bzw. der Folge von Ultraschallschweißstellen 30 ist ebenfalls im Wesentlichen entlang dieser ersten Richtung 32. Wie aus Figur 1 ersichtlich ist, ist der Cuffsockel 28 jedoch wenigstens bereichsweise nicht gerade und parallel zu der ersten Richtung 32, sondern sie erstreckt sich kurven- oder bogenförmig und hat somit auch eine Komponente in Richtung einer zur ersten Richtung senkrechten zweiten Richtung 34 (Querrichtung des Hygieneartikels). Das von den Ultraschallschweißstellen 30 zumindest teilweise gebildete Fügemuster 36 wird nachfolgend noch im Einzelnen beschrieben werden. Jedenfalls bilden die beim bestimmungsgemäßen Gebrauch unlösbar miteinander verbundenen Vliesstoffkomponenten und/oder Folienkomponenten einen Vliesstoffverbund 38.

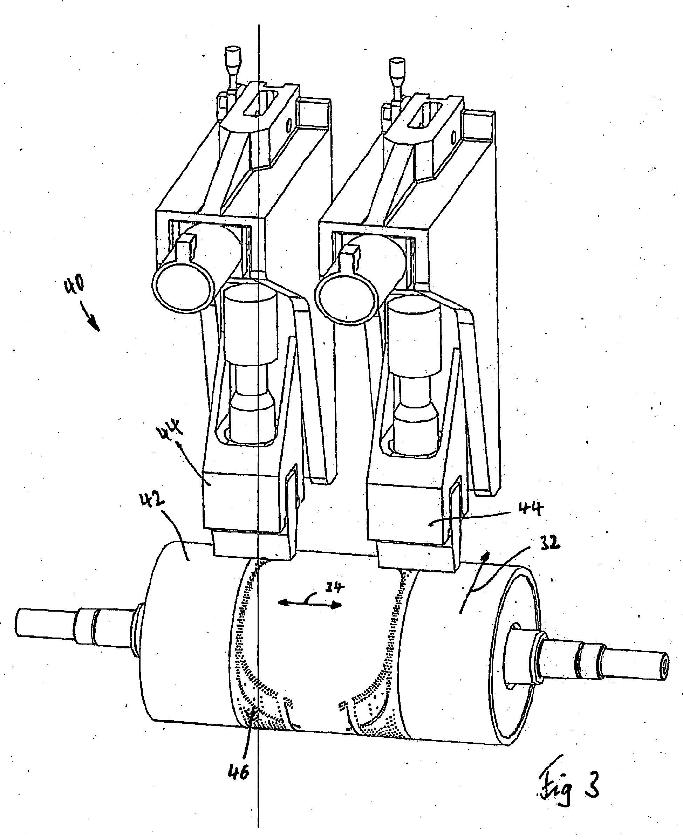

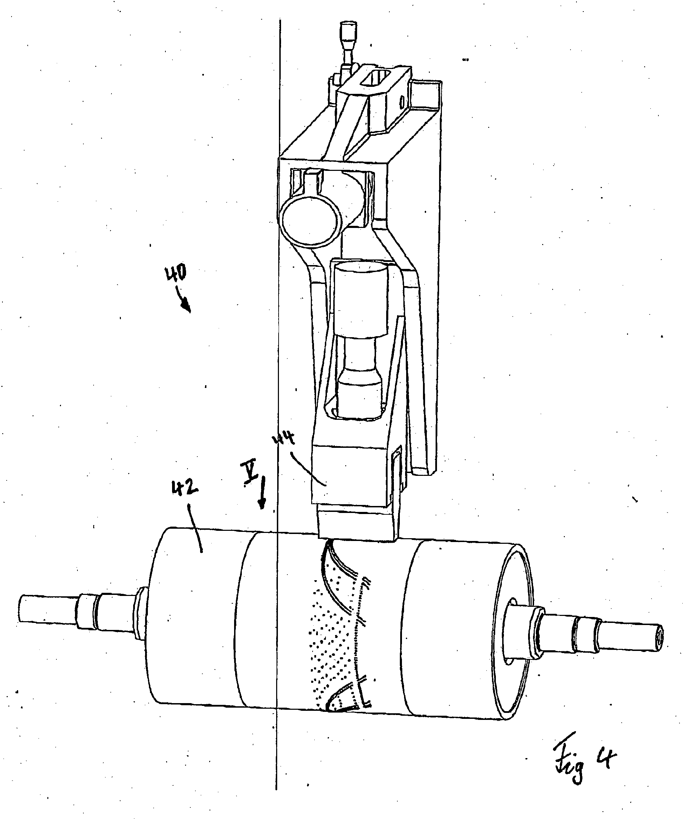

Die schematischen Darstellung einer in Figuren 3, 4 insgesamt mit dem Bezugszeichen 40 bezeichneten Ultraschallschweißvorrichtung verdeutlicht eine in der Maschinenrichtung oder ersten Richtung 32 abrollende Konturwalze 42 und eine im Ultraschallbereich anregbare Sonotrode 44. Die Ultraschallschweißvorrichtung 40 ist in eine schnelllaufende Maschine zur Herstellung moderner Hygieneartikel integrierbar. Figur 3 verdeutlicht eine Ultraschallschweißvorrichtung mit zwei Sonotroden 44 zur Herstellung der Schweißverbindung zweier beidseitiger Cuffelemente, während die Vorrichtung nach Figur 4 nur eine Sonotrode 44 offenbart und nur für die Herstellung der Ultraschallschweißverbindung bei einem Cuffelement vorgesehen ist. Eine entsprechende weitere Konturwalze und Sonotrode für die andere Hälfte könnte vorgeordnet, nachgeordnet oder parallel vorgesehen sein. Ebenso wäre es denkbar, dass bei der Ausführungsform nach Figur 3 anstelle zweier nebeneinander angeordneter Sonotroden eine einzige in der zweiten Richtung 34 durchgehende Sonotrode vorgesehen wird.

Im Zuge der Herstellung des in Figuren 1 und 2 dargestellten Hygieneartikels würde beispielsweise in den Spalt der durch Sonotrode und Konturwalze gebildeten Ultraschallschweißvorrichtung nach Figur 3 ein Cuffelement 14 und das Topsheet 4 in übereinander angeordneter Konfiguration eingeführt werden, um diese Komponenten zur Bildung des Vliesstoffverbundes aneinanderzufügen und anschließend auf den Saugkörper 8 zu platzieren.

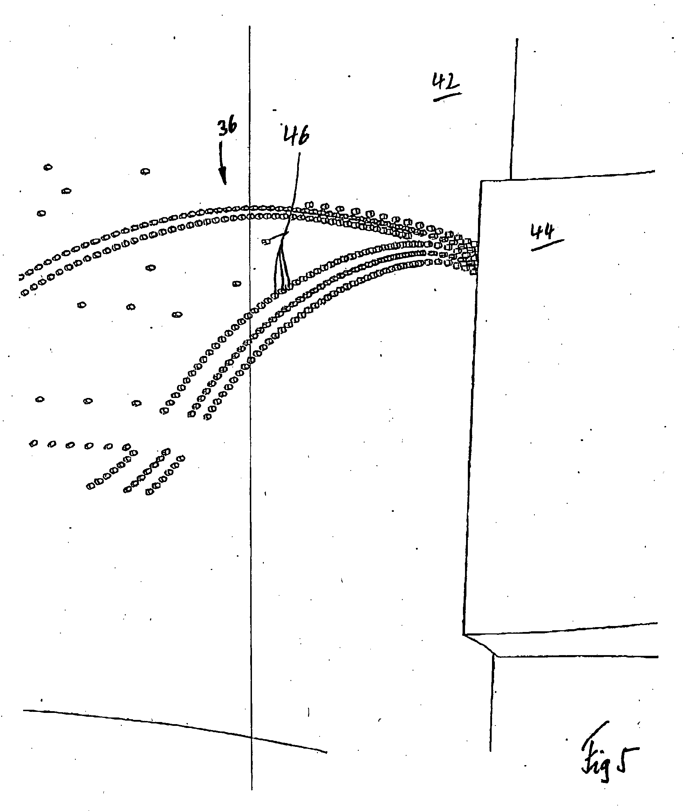

Man erkennt des Weiteren auf dem Umfang der Konturwalze 42 eine Vielzahl von Erhebungen 46, welche entsprechend dem herzustellenden Fügemuster 36 angeordnet und ausgebildet sind. Figur 5 zeigt eine Draufsicht auf die Oberfläche der Konturwalze 42 gesehen in Richtung des Pfeils V in Figur 4. Man erkennt im Einzelnen die Erhebungen 46 auf der Konturwalze 42 und das durch sie dann in entsprechend identischer Weise beim Abrollen gegenüber den zugeführten Flachmaterialbahnen gebildete Fügemuster 36. Man erkennt eine linienförmige oder reihenförmige Anordnung von Erhebungen 46, welche Ultraschallschweißstellen 30 des späteren Cuffsockels 28 bilden. Man erkennt außerdem, dass der Abstand der Erhebungen 46 voneinander und die Anzahl der Erhebungen 46 pro Flächeneinheit auf der Walzenoberfläche 42 variieren, damit die einzelnen nachfolgend beschriebenen Bedingungen für das entstehende Fügemuster 36 erfüllt werden.

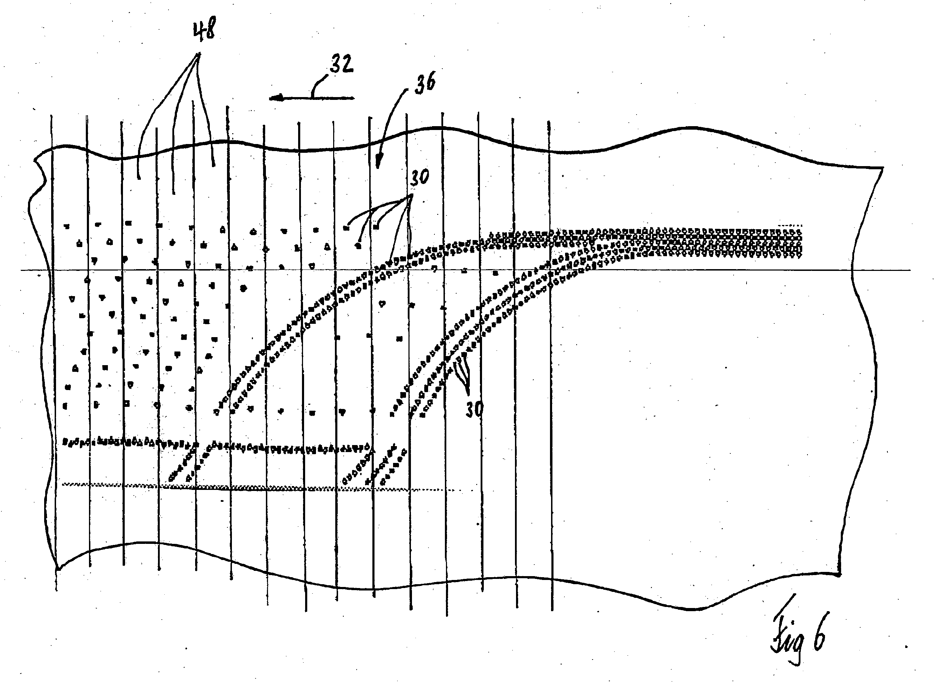

Figur 6 zeigt eine Draufsicht auf ein Fügemuster in schematischer Darstellung zur Verdeutlichung der gedanklichen Unterteilung des Fügemusters in 5 mm breite Abschnitte 48 in der Längsrichtung 32. Die Abschnitte 48 schließen sich also in der ersten Richtung 32 aneinander an. Innerhalb eines jeden 5 mm-Abschnitts 48 sind eine Anzahl von Ultraschallschweißstellen 30 vorgesehen, die für jeden 5 mm-Abschnitt 48 einen Flächenanteil $A_i$ verprägter bzw. verschweißter Fläche bilden. Sind N solcher 5 mm-Abschnitte 48 vorgesehen, die sich gedanklich in der ersten Richtung 32 vom Beginn bis zum Ende des Ultraschallfügemusters 36 anordnen, so lässt sich ein Mittelwert $\bar{A}$ folgendermaßen ermitteln:

$$\overline{A} = \frac{\sum Ai}{N}$$

Es lässt sich ebenfalls eine Größe s aus der Summe der Abweichungsquadrate der einzelnen $A_i$ von diesem Mittelwert $\overline{A}$ nach folgender Formel ermitteln:

$$s = \sqrt{\frac{1}{N-1}\sum_{i=1}^{N}(Ai - \overline{A})^2}$$

Hieraus lässt sich dann für das gesamte Fügemuster 36 bzw. die gedachte Unterteilung in N 5 mm-Abschnitte 48 ein Index I der Schwankung der von den Ultraschallschweißstellen erfassten Fläche in der ersten Richtung 32 folgendermaßen angeben:

$$I = \frac{s}{\overline{A}} \times 100[\%]$$

Erfindungsgemäß wurde festgestellt, dass dieser Index der Schwankung der von den Ultraschallschweißstellen 30 erfassten Fläche in der ersten Richtung 32 betrachtet höchstens 40 % betragen sollte.

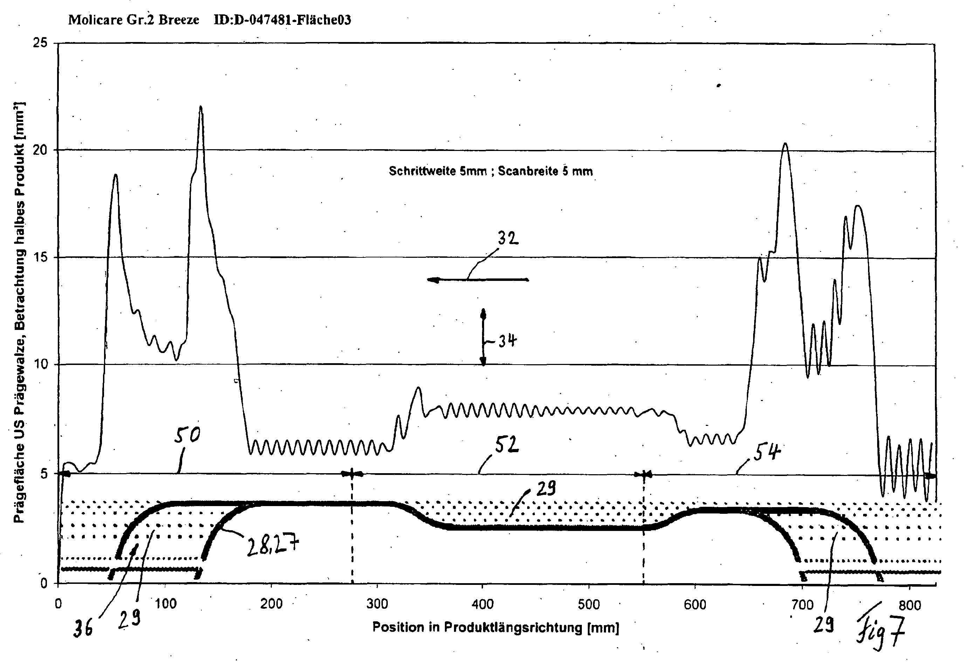

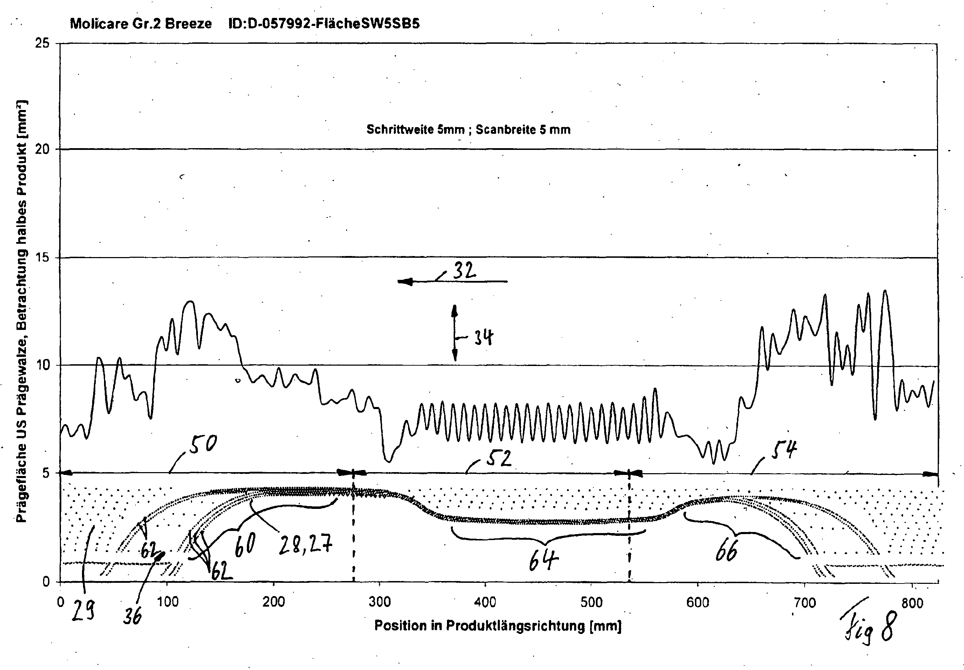

Nachfolgend werden anhand der Figuren 7 und 8 zwei verschiedene Fügemuster aus diskreten Ultraschallschweißstellen untersucht. Ersichtlicherweise handelt es sich um ein Fügemuster zur Fixierung aus Figuren 1 und 2 ersichtliche Cuffelemente 14, d. h. der Vliesstoffkomponente 16 dieser Cuffelemente 14 an eine weitere Vliesstoff- oder Folienkomponente 10 eines Hygieneartikels. Es wurde das Fügemuster eines der beiden Cuffelemente 14 des Hygieneartikels betrachtet.

Figur 7 zeigt im unteren Bereich in Erstreckung der Abszisse die Ausbildung des betrachteten Fügemusters von Ultraschallschweißpunkten, die in einem ersten Bereich 27 einen geringen Abstand und in einem zweiten Bereich 29 einen größeren Abstand voneinander aufweisen. Die Erstreckung des Fügemusters in der ersten Richtung (Maschinenrichtung) beträgt 820 mm, woraus sich 164 5 mm-Abschnitte ergeben. Oberhalb des angedeuteten Fügemusters sind diejenigen Flächenanteile der jeweiligen verschweißten Fläche innerhalb eines betreffenden 5 mm-Abschnitts aufgetragen und zu einer Kurve verbunden. Es handelt sich also um 164 Flächenanteile. $A_i$, einen für jeden 5 mm-Abschnitt. Man erkennt aufgrund des verhältnismäßig geringen Krümmungsradius der mit hoher Rasterdichte der Ultraschallschweißstellen ausgebildeten Cuffsockeln 28 eine sehr hohe Schwankung der Flächenanteile in der ersten Richtung 32. Dies führt aber zu den eingangs geschilderten Problemen. Der dargestellte Verlauf der Flächenanteile $A_i$ führt zu einem Mittelwert von 9,01 mm$^2$

betrachtet über alle N = 164 5mm-Abschnitte. Dies führt zu einem Maß s von 3,85 und zu einem Index I von 42,7 %.

Zerlegt man das Fügemuster 36 in einen Anfangsabschnitt 50, einen Mittelabschnitt 52 und einen Endabschnitt 54, die sich jeweils über ein Drittel der Erstreckung des Fügemusters 36 in der Längsrichtung erstrecken, so gelangt man für diese Abschnitte 50, 52, 54 bei isolierter Auswertung der darin enthaltenen N/3 5 mm-Abschnitte zu folgenden Werten:

Anfangsabschnitt : $\overline{A}$ = 9,55, s = 4,66, I = 48,8 %
Mittelabschnitt 52: $\overline{A}$ = 7,62, s = 0,71, I = 9,3 %
Endabschnitt 54: $\overline{A}$ = 9,90, s = 4,49, I = 45,3 %.

Aufgrund dieses Werts des Index I der Schwankung der von den Ultraschallschweißstellen erfassten Fläche wäre ein Hygieneartikel mit dem in Figur 7 dargestellten Fügemuster 36 nicht erfindungsgemäß.

Eine erfindungsgemäße Ausbildung eines Fügemusters 36 ist in Figur 8 dargestellt. Man gelangt bei isolierter Auswertung der in den Abschnitten 50, 52, 54 enthaltenen N/3 5 mm-Abschnitte zu folgenden Werten:

Anfangsabschnitt: $\overline{A}$ = 9,56, s = 1,67, I = 17,5 %

Mittelabschnitt 52: $\overline{A}$ = 7,34, s = 0,91, I = 12,4 %

Endabschnitt 54: $\overline{A}$ = 9,25, s = 2,33, I = 25,1 %.

Man erkennt aus der Figur 8, dass der Anteil der geschweißten Flächen $A_i$ in der ersten Richtung 32 sehr viel weniger variiert als bei dem Fügemuster nach Figur 7. Dies wird erreicht durch eine im Hinblick auf einen möglichst geringen Index der Schwankung der von den Ultraschallschweißstellen erfassten Fläche. Insbesondere erkennt man, dass ein erstes Teilfügemuster 60, welches einen Teil eines Cuffsockels 28 bildet und bogenförmig gekrümmt verläuft, aus einer Schar von mehreren Kurven 62 gebildet ist. Der Begriff Kurve wird hier verwendet für eine Reihe aufeinander folgender diskreter Ultraschallschweißstellen 30 (so wie dies auch aus Figur 5 ersichtlich ist). Diese Schar von Kurven 62 ist beispielsweise in einem Bereich von ca. 200 bis 270 mm in engem Abstand und parallel sowie im Wesentlichen in der ersten Richtung 32 verlaufend angeordnet. In dem daran anschließenden Bereich (von ca. 200 bis ca. 50 mm) sind die Kurven 62 bogenförmig gekrümmt, und ihr Abstand nimmt in der ersten Richtung 32 zu. Auf diese Weise wird erfindungsgemäß erreicht, dass die Flächenanteile $A_i$ der 5 mm-Abschnitte in der ersten Richtung 32 nicht so stark variieren, wie beispielsweise bei dem Fügemuster nach Figur 7. Insgesamt resultiert ein sehr viel geringerer Index der Schwankung der von den Ultraschallschweißstellen erfassten Fläche.

Es ist unmittelbar ersichtlich, dass es mehrere Möglichkeiten gibt, zu erreichen, dass die Variation der Flächenanteile $A_i$ der 5 mm-Abschnitte in der ersten Richtung 32 nicht so stark ist. Eine erste Möglichkeit ist, bei gekrümmt verlaufenden Teilfügemustern den Krümmungsradius möglichst groß zu halten, also keine starken Krümmungen zuzulassen. Eine weitere Möglichkeit, die in Figur 8 gezeigt ist, ist die Ausbildung von gekrümmten Teilfügemustern in Form mehrerer Kurven oder Reihen von Ultraschallschweißstellen, deren Abstand zueinander insbesondere variierbar ist. Ebenso könnte aber auch der Abstand der einzelnen Ultraschallschweißstellen, insbesondere einer Reihe, variiert werden. Ebenso könnte, insbesondere zusätzlich, die Fläche der einzelnen Ultraschallschweißstellen bzw. Erhebungen auf der Konturwalze variiert werden. Außerdem kann die Anordnung und der Abstand der Ultraschallschweißpunkte in einem zweiten Bereich, also dort wo die Ultraschallschweißstellen einen größeren Abstand von einander aufweisen, variiert werden.

Schließlich zeigt Figur 8 ein zweites Teilfügemuster 64, welches im Wesentlichen geradlinig und in der ersten Richtung 32 erstreckt ist. Von einer solchen Erstreckung im Wesentlichen in der ersten Richtung 32 ist dann die Rede, wenn über eine Länge von etwa 200 mm in der ersten Richtung 32 eine Ausdehnung in der zweiten Richtung 34 von maximal 5 mm festellbar ist. Hieran schließt sich ein drittes Teilfügemuster 66 an, welches wie das erste Teilfügemuster 60 bogenförmig gekrümmt verläuft und eine Schar von mehreren Kurven 62 umfasst. Es weist somit auch eine Komponente in der zweiten Richtung 34 auf.

**Patentansprüche**

1. Hygieneartikel (2) zum einmaligen Gebrauch, wie Inkontinenzwindel, Inkontinenzvorlage, mit einem Vliesstoffverbund (38), wobei der Vliesstoffverbund (38) mindestens eine erste Vliesstoffkomponente (16) und eine weitere Vliesstoff- oder Folienkomponente (10, 12) aufweist und wobei die erste Vliesstoffkomponente (14) an der weiteren Vliesstoff- oder Folienkomponente (10, 12) zumindest bereichsweise durch ein Fügemuster (36), das diskrete Ultraschallschweißstellen (30) umfasst, zur Bildung des Vliesstoffverbunds (38) festgelegt ist, wobei der Hygieneartikel ein zumindest bereichsweise flüssigkeitsdurchlässiges Topsheet (4), ein zumindest bereichsweise flüssigkeitsdichtes Backsheet (6) und einen dazwischen angeordneten Körperflüssigkeiten absorbierenden Speicherkern (8) und beidseitig mindestens ein eine Auslaufsperre bildendes, im Wesentlichen in der ersten Richtung (32) verlaufendes, zumindest abschnittsweise aufstehendes Cuffelement (14) umfässt, wobei das Cuffelement (14) die erste Vliesstoffkomponente (16) umfasst, und wobei die weitere Vliesstoff- oder Folienkomponente (10, 12) das Topsheet (4) oder das Backsheet (6) ist, so dass das Cuffelement (14) mit seiner ersten Vliesstoffkomponente (16) an dem Topsheet (4) oder Backsheet (6) durch das Fügemuster (36) festgelegt ist, und wobei das Fügemuster (36) sich in einer ersten Richtung (32) und senkrecht dazu in einer zweiten Richtung (34) erstreckt und die. Erstreckung in der ersten Richtung (32) größer ist als in der zweiten Richtung (34), dass ein Cuffsockel (28) des Cuffelements (14) zumindest bereichsweise von einem ersten Teilfügemuster (60), einem zweiten im wesentlichen geradlinig verlaufenden Teilfügemuster (64) und von einem dritten nicht geradlinig verlaufenden Teilfügemuster (66) gebildet ist, wobei das zweite Teilfügemüster (64) in der ersten Richtung (32) zwischen dem ersten (60) und dem dritten Teilfügemuster (66) angeordnet ist und wobei sich so eine Ausbildung des Cuffsockels (28) ergibt, der in einem vorderen Bereich und in einem hinteren Bereich des Artikels quer zur Längsrichtung bogenförmig geschwungen hierzu verläuft und in einem dazwischen angeordneten Bereich im Wesentlichen geradlinig und insbesondere parallel zur ersten Richtung (32) verläuft, und dass der Abstand der diskreten Ultraschallschweißstellen (30) voneinander in einem ersten Bereich (27) des Fügemusters (36) geringer ist als in einem zweiten Bereich (29) des Fügemusters (36), wobei der erste Bereich (27) zumindest einen Teil des ersten (60) und des zweiten (64) und des dritten (66) Teilfügemusters (60, 64, 66) des Cuffsockels (28) bildet, und wobei der Index (I) der Schwankung der von den Ultraschallschweißstellen erfassten Fläche in der ersten Richtung (32) höchstens 40% beträgt.

2. Artikel nach Anspruch 1, **dadurch gekennzeichnet, dass** der Index (I) der Schwankung der von den Ultraschalischweißstellen (30) erfassten Fläche höchstens 35%, insbesondere höchstens 30 %, weiter insbesondere höchstens 25% beträgt.

3. Artikel nach einem der vorstehenden Ansprüche, **dadurch gekennzeichnet, dass** das Fügemuster (36) in der ersten Richtung (32) betrachtet virtuell in drei gleich lange Abschnitte, nämlich einen Anfangsabschnitt (50), einen sich daran anschließenden Mittelabschnitt (52) und einen sich an den Mittelabschnitt (52) anschließenden Endabschnitt (54) ünterteilbar ist und dass der Index (I) der Schwankung der von den Ultraschallschweißstellen erfassten Fläche des Anfangsabschnitts (50) und/oder des Endabschnitts (54) des Fügemuster höchstens 40 %, insbesondere höchstens 35%, weiter insbesondere höchstens 30%, weiter insbesondere höchstens 25% beträgt.

4. Artikel nach einem der vorstehenden Ansprüche, **dadurch gekennzeichnet, dass** der Abstand einzelner (diskreter) Ultraschallschweißstellen (30) voneinander in dem ersten Bereich (27) 0,1 - 2,5 mm, insbesondere 0,2 -2,3 mm und weiter insbesondere 0,2 - 1,5 mm beträgt und in dem zweiten Bereich (29) 2 - 20 mm, insbesondere 3 - 15 mm, weiter insbesondere 4 - 10 mm beträgt.

5. Artikel nach einem der vorstehenden Ansprüche, **dadurch gekennzeichnet, dass** die Flächendichte der Ultraschallschweißstellen (30) in dem ersten Bereich (27) größer ist als in dem zweiten Bereich (29).

6. Artikel nach einem der vorstehenden Ansprüche, **dadurch gekennzeichnet, dass** das erste (60) und/oder dritte (66) Teilfügemuster eine Schar aus mindestens zwei insbesondere mindestens drei Kurven (62), weiter insbesondere mindestens vier, weiter insbesondere mindestens fünf Kurven (62) umfasst.

7. Verfahren zur Herstellung eines Hygieneartikels (2) zum einmaligen Gebrauch, wie Inkontinenzwindel, Inkontinenzvorlage, nach einem der vorstehenden Ansprüche, **dadurch gekennzeichnet, dass** der Index (I) der Schwankung der von den Ultraschallschweißstellen (30) erfassten Fläche höchstens 40 % beträgt.

8. Verfahren nach Anspruch 7, **dadurch gekennzeichnet, dass** die Ultraschallschweißvorrichtung (40) eine (feststehende) Sonotrode (44) und eine Konturwalze (42) umfasst.

9. Verfahren nach Anspruch 7 oder 8, **dadurch gekennzeichnet, dass** Verschweißen der Komponenten mit einer Bahngeschwindigkeit von 100 - 1000 m/min durchgeführt wird.

**Claims**

1. Hygiene article (2) for single use, such as an incontinence diaper, incontinence pad, including a composite nonwoven fabric (38) wherein the composite nonwoven fabric (38) comprises at least a first nonwoven fabric component (16) and another further nonwoven fabric or film component (10, 12), and wherein the first nonwoven fabric component (16) is attached to the other nonwoven fabric or film component (10, 12) in at least some areas by means of a joining pattern (36) that includes discrete ultrasonic welding points (30) in order to form the composite nonwoven fabric (38), wherein the hygiene article comprises a topsheet (4) that is permeable at least in some areas, a backsheet (6) that is impermeable at least in some areas, and an absorbent core (8) that absorbs body fluids disposed between the two and comprises on both sides at least one cuff element (14) forming a leakage barrier, extending essentially in the first direction (32) and raising at least in some sections, wherein the cuff element (14) comprises the first nonwoven fabric component (16) and wherein the further nonwoven fabric or film component (10, 12) is the topsheet (4) or the backsheet (6), so that the cuff element (14) with its first nonwoven fabric component (16) is attached by the joining pattern (36) to the topsheet (4) or backsheet (6), and wherein the joining pattern (36) extends in a first direction (32) and in a second direction (34) that runs perpendicular thereto, the extension in the first direction (32) being longer than the extension in the second direction (34), and wherein a cuff base (28) of the cuff element (14) at least in some sections is formed by a first partial joining pattern (60), a second substantially rectilinear partial joining pattern (64) and a third non-rectilinear partial joining pattern (66) and wherein in the first direction (32) the second partial joining pattern (64) is disposed between the first (60) and the third partial joining pattern (66), and wherein accordingly a form of the cuff base (28) results, which in a front area and in a rear area of the article extends transverse to the longitudinal direction in a curved shape of an arc with respect to the longitudinal direction and in an intermediate area is substantially rectilinear and, in particular, parallel to the first direction (32), and that the distance between the discrete ultrasonic welding points (30) from each other in a first area (27) of the joining pattern (36) is less than that in a second area (29) of the joining pattern (36), wherein the first area (27) forms at least a part of the first (60) and second (64) and third (66) partial joining pattern (60, 64, 66) of the cuff base (28), and wherein the index (I) of variation of the area seized by the ultrasonic welding points in the first direction (32) is at most 40%.

2. Article according to the claim 1, **characterized in that** the index (I) of variation of the area seized by the ultrasonic welding points (30) is at most 35%, in particular, at most 30 %, further, in particular, at most 25%.

3. Article according to any one of the previous claims, **characterized in that** the joining pattern (36) viewed in the first direction (32) can be conceptually subdivided in the first direction in three sections of equal length, that is, a start section (50), an adjoining center section (52), and a section (54) adjoining the center section (52) and that the index (I) of variation of the area seized by the ultrasonic welding points of the start section (50) and/or of the end section (54) of the joining pattern is at most 40 %, in particular, at most 35%, further, in particular, at most 30%, further, in particular, at most 25%.

4. Article according to any one of the previous claims, **characterized in that** the distance between individual (discrete) ultrasonic welding points (30) in the first area (27) is 0.1 to 2.5 mm, in particular, 0.2 to 2.3 mm and further, in particular, 0.2 to 1.5 mm and in the second area (29), is 2 to 20 mm, in particular, 3 to 15 mm, further, in particular, 4 to 10 mm.

5. Article according to any one of the previous claims, **characterized in that** the density per unit area of the ultrasonic welding points (30) in the first area (27) is greater than that in the second area (29).

6. Article according to any one of the previous claims, **characterized in that** the first (60) and/or third (66) partial joining pattern comprises a set of at least two, in particular, at least three curves (62), further, in particular, at least four, further, in particular, at least five curves (62).

7. Method for the production of a hygiene article (2) for single use such as incontinence diaper, incontinence pad, according to any one of the previous claims, **characterized in that** the index (I) of variation of the area seized by the ultrasonic welding points (30) is no more than 40 %.

8. Method according to claim 7, **characterized in that** the ultrasonic welding device (40) comprises a (stationary) sonotrode (44) and a contour roller (42).

9. Method according to claim 7 or 8, **characterized in that** welding of the components is performed at a path velocity of 100 to 1000 m/min.

**Revendications**

1. Article hygiénique (2) à usage unique, telle que couche pour l'incontinence, protection pour l'incontinence, comprenant un assemblage en non-tissé (38), dans lequel ledit assemblage en non-tissé (38) présente au moins un premier composant en non-tissé (16) et un autre composant en non-tissé ou en film (10, 12) et dans lequel le premier composant en non-tissé (14) est fixé sur ledit autre composant en non-tissé ou en film (10, 12), au moins par zones, par un motif d'assemblage (36) qui comprend des points discrets de soudage par ultrasons (30), pour former ledit assemblage en non-tissé (38), dans lequel ledit article hygiénique comprend une feuille supérieure (4) perméable au liquide au moins par zones, une feuille arrière (6) étanche au liquide au moins par zones et un noyau accumulateur (8) disposé entre celles-ci et absorbant des fluides corporels ainsi que, de part et d'autre, au moins un élément barrière (14) qui forme une barrière à l'écoulement, s'étend pour l'essentiel dans la première direction (32) et est dressé au moins par sections, dans lequel ledit élément barrière (14) comprend le premier composant en non-tissé (16) et dans lequel l'autre composant en non-tissé ou en film (10, 12) est ladite feuille supérieure (4) ou ladite feuille arrière (6) de sorte que l'élément barrière (14) avec son premier composant en non-tissé (16) est fixé à la feuille supérieure (4) ou feuille arrière (6) par ledit motif d'assemblage (36), et dans lequel le motif d'assemblage (36) s'étend dans une première direction (32) et perpendiculairement à celle-ci dans une deuxième direction (34) et l'extension dans la première direction (32) est plus importante que dans la deuxième direction (34), qu'un socle de barrière (28) dudit élément barrière (14) est formé, au moins par zones, par un premier motif partiel d'assemblage (60), par un deuxième motif partiel d'assemblage (64) s'étendant pour l'essentiel de manière rectiligne et par un troisième motif partiel d'assemblage (66) ne s'étendant pas de manière rectiligne, dans lequel le deuxième motif partiel d'assemblage (64) est disposé dans la première direction (32) entre les premier (60) et troisième (66) motifs partiels d'assemblage, et dans lequel il en résulte ainsi une configuration dudit socle de barrière (28) qui, dans une zone avant et dans une zone arrière de l'article, transversale à la direction longitudinale, s'étend de manière incurvée en arc par rapport à ceci et, dans une zone disposée entre celles-ci, d'une manière pour l'essentiel rectiligne et en particulier parallèlement à la première direction (32), et que la distance séparant les points discrets de soudage par ultrasons (30) les uns des autres est plus petite dans une première zone (27) du motif d'assemblage (36) que dans une deuxième zone (29) du motif d'assemblage (36), dans lequel la première zone (27) forme au moins une partie des premier (60) et deuxième (64) et troisième (66) motifs partiels d'assemblage (60, 64, 66) du socle de barrière (28), et dans lequel l'indice (I) de la variation de la surface couverte par les points de soudage par ultrasons, dans la première direction (32), est de 40 % tout au plus.

2. Article selon la revendication 1, **caractérisé par le fait que** l'indice (I) de la variation de la surface couverte par les points de soudage par ultrasons (30) est de 35 % tout au plus, en particulier de 30 % tout au plus, encore en particulier de 25 % tout au plus.

3. Article selon l'une quelconque des revendications précédentes, **caractérisé par le fait que** le motif d'assemblage (36), vu dans la première direction (32), peut être divisé virtuellement en trois sections de même longueur, à savoir une section initiale (50), une section centrale (52) qui suit celle-ci et une section terminale (54) qui suit ladite section centrale (52), et que l'indice (I) de la variation de la surface de la section initiale (50) et/ou de la section terminale (54) du motif d'assemblage, qui est couverte par les points de soudage par ultrasons, est de 40 % tout au plus, en particulier de 35 % tout au plus, encore en particulier de 30 % tout au plus, encore en particulier de 25 % tout au plus.

4. Article selon l'une quelconque des revendications précédentes, **caractérisé par le fait que** la distance séparant des points de soudage par ultrasons (30) individuels (discrets) les uns des autres, est comprise entre 0,1 et 2,5 mm, en particulier entre 0,2 et 2,3 mm et, encore en particulier, entre 0,2 et 1,5 mm dans la première zone (27) et est comprise entre 2 et 20 mm, en particulier entre 3 et 15 mm, encore en particulier entre 4 et 10 mm dans la deuxième zone (29).

5. Article selon l'une quelconque des revendications précédentes, **caractérisé par le fait que** la densité surfacique des points de soudage par ultrasons (30) est plus importante dans la première zone (27) que dans la deuxième zone (29).

6. Article selon l'une quelconque des revendications précédentes, **caractérisé par le fait que** le premier (60) et/ou le troisième (66) motif partiel d'assemblage comprend une famille d'au moins deux, en particulier d'au moins trois courbes (62), encore en particulier d'au moins quatre, encore en particulier d'au moins cinq courbes (62).

7. Procédé de fabrication d'un article hygiénique (2) à usage unique, telle que couche pour l'incontinence, protection pour l'incontinence, selon l'une quelconque des revendications précédentes, **caractérisé par le fait que** l'indice (I) de la variation de la surface couverte par les points de soudage par ultrasons (30) est de 40 % tout au plus.

8. Procédé selon la revendication 7, **caractérisé par le fait que** le dispositif de soudage par ultrasons (40) comprend une sonotrode (fixe) (44) et un rouleau à contour (42).

9. Procédé selon la revendication 7 ou 8, **caractérisé par le fait que** le soudage des composants est réalisé à une vitesse de bande comprise entre 100 et 1000 m/min.

Fig 1

Fig 2

Fig 3

Fig 4

36  46  42  44

Fig 5

Fig 6

Molicare Gr.2 Breeze    ID:D-047481-Fläche03

Schrittweite 5mm ; Scanbreite 5 mm

*y-axis:* Prägefläche US Prägewalze, Betrachtung halbes Produkt [mm²]

*x-axis:* Position in Produktlängsrichtung [mm]

Fig 7

EP 2 063 842 B1

18

EP 2 063 842 B1

Fig 8

Molicare Gr.2 Breeze   ID:D-057992-FlächeSW5SB5

Schrittweite 5mm ; Scanbreite 5 mm

Prägefläche US Prägewalze, Betrachtung halbes Produkt [mm²]

Position in Produktlängsrichtung [mm]

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 200405668 A